# EUROPEAN PATENT APPLICATION

(11) **EP 3 854 379 A1**
(43) Date of publication of application: **28.07.2021**
(21) Application number: 19790114.3
(22) Date of filing: 27.08.2019
(51) Int. Cl.: A61K 8/9789, A61Q 19/02, A61Q 19/08, A61K 36/23, A61P 17/00, A23L 33/105

(54) **COSMETIC COMPOSITION COMPRISING CENTELLA ASIATICA ADVENTITIOUS ROOT EXTRACT AS EFFECTIVE INGREDIENT FOR SKIN WHITENING AND WRINKLE REDUCTION**

(30) Priority: 19.09.2018 KR 20180112354
(71) Applicant: Dong Kook Pharm. Co., Ltd, Gangnam-gu Seoul 06175 (KR)
(72) Inventor: HWANG, Young, 28334 Chungcheongbuk-do (KR); HAN, Su Min, 28117 Chungcheongbuk-do (KR); KIM,Young Eun, 30101 Sejong-si (KR)
(74) Representative: V.O.
(86) International application number: PCT/KR2019/010948
(87) International publication number: WO 2020/060060

(57) **Abstract**

The present invention relates to a cosmetic composition for skin whitening and wrinkle improvement comprising an adventitious root extract of *Centella asiatica* as an effective ingredient, and as the adventitious root extract of *Centella asiatica* of the present invention has an excellent anti-inflammation and antioxidant activity without any cytotoxicity and also has an excellent effect of inhibiting the melanin pigmentation and matrix metalloprotease and enhancing the production of procollagen as a precursor of collagen, it can be advantageously used as a cosmetic, a preparation for external application on skin, or the like for skin whitening and wrinkle improvement.

## Description

### TECHNICAL FIELD

The present invention relates to a cosmetic composition for skin whitening and wrinkle improvement comprising adventitious root extract of *Centella asiatica* as an effective ingredient.

### BACKGROUND ART

As aged population increases, the anti-oxidation for having skin care and preventing skin deterioration, which is an indicator of skin aging, receives increasing attention. Occurrence of skin wrinkle and reduced antioxidant activity in a living body, which are caused by aging, can be taken as a health indicator. Depending on the cause, skin aging can be classified into intrinsic aging and extrinsic aging. It is known that the intrinsic aging is caused by a change in physiological function of epidermis and dermis which occurs with aging, while the extrinsic aging is caused by a change in physiological function of skin which occurs due to an environmental factor like air contamination, exposure to UV radiation, and stress. The common mechanism between those agings can be explained by increased free radicals in a living body as caused by continuous exposure to an oxidative stress, enhanced activity of elastase which decomposes elastin as connective tissues in dermis, and enhanced activity of hyaluronidase which decomposes hyaluronic acid. Namely, the skin aging can be explained as a process of having skin wrinkle as elastin and hyaluronic acid are decomposed so as to yield breakdown of dermis and dry skin. In addition, being involved in the process of forming melanin, free radicals in a living body can be also a cause of yielding dark spots and freckles by activating tyrosinase.

In everyday life, people are constantly exposed to the harm caused by active oxygen (i.e., reactive oxygen), and, as the harm gradually accumulates with aging, the antioxidant activity is subsequently reduced. At normal physical conditions, the active oxygen is removed by an antioxidant protection system via a pathway including an enzyme reaction (e.g., superoxide dismutase (SOD)) and a non-enzyme reaction (e.g., polyphone and falconoid). Thus, for having exhibition of the antioxidant activity, it is necessary to activate an antioxidant enzyme reaction like SOD or intake a non-enzyme material which directly shows the antioxidant activity.

Studies have been actively made on a mechanism relating to skin health and anti-oxidation. Due to an advancement in the level of study and techniques, various studies are being made to develop a material having biochemical efficacy and effect. In particular, a material derived from plants has been used for a long period of time as it is acknowledged with an excellent safety. In South Korea, development of a functional material which contains plant and herbal medicine components that are used either in traditional medicine or oriental medicine is actively made. In particular, in the field of cosmetics, great attention is paid to plant materials for the purpose of preventing skin aging or hair loss, having anti-oxidation or the like by using useful materials derived from plants (e.g., carotenoids, phenol compounds, vitamins, or the like).

However, plants have slow growth rate, and useful materials contained in plants are present in a small amount only in a specific plant organ, and type and content of second metabolites vary depending on plant variety. Furthermore, plants are characterized in that, even in the same variety, the productivity varies depending on seasons, location, climate, culture conditions, part of plant, or the like. Furthermore, due to Nagoya Protocol became effective in recent years, import of foreign materials is now more difficult than ever. As such, many studies have been made to produce various plant-based useful materials (i.e., second metabolites) by biotechnology techniques. Among them, the most effective production method is a plant cell culture method. This method allows effective production of the second metabolites by using culture medium, culture conditions, and a treatment with various elicitors. Furthermore, as it allows stable and continuous production of a large amount of a useful material, which is originally produced by a plant, without being limited by natural environments (e.g., pesticides, heavy metal contamination, seasonal climate change, or the like), the plant cell culture method is believed to have commercial importance.

*Centella asiatica* (*Centella asiatica* (L.) Urban) is a perennial plant belonging to *Umbelliferae,* and it has scale-like leaves while roots are grown from a node and main stalks grow laterally. Naturally growing mostly in a region with high temperature and high humidity, *Centella asiatica* is found in Islands of Madagascar of Africa, coastal areas of Indian Ocean, and Malaysian regions. In South Korea, natural existence of *Centella asiatica* is extremely limited to Jeju Island and islands of the southern region, which belong to the warm temperature zone. An extract of *Centella asiatica* contains asiaticoside, madecassoside, asiatic acid, madecassic acid, or the like, and thus it is very useful for wound healing, and, as being effective for dementia, stomach disorder, or the like, it is used as a raw material of an agent for treating disorders.

Meanwhile, in Korean Patent Registration No. 1739422, "Composition for improving skin conditions and preventing or treating skin diseases containing *Centella asiatica* originating from plant factory" is described, and in Korean Patent Registration No. 1881417, "Cosmetic composition comprising menthol, extracts of *Ginko biloba* leaf, *Aloe barvadensis, Camellia sinesis, Centella asiatica,* and *Polygonum multiflorum"* is disclosed. However, there is no description relating to the cosmetic composition of the present invention for skin whitening and wrinkle improvement which comprises an adventitious root extract of *Centella asiatica* as an effective ingredient.

### DETAILED DESCRIPTION OF THE INVENTION

### TECHNICAL PROBLEMS TO BE SOLVED

The present invention is devised under the circumstances described in the above, and the inventors of the present invention found that, compared to an extract of *Centella asiatica,* an adventitious root extract of *Centella asiatica* has a significantly more excellent effect of inhibiting the production of melanin and matrix metalloprotease (hereinbelow, MMP) as well as an excellent effect of inhibiting production of nitric oxide (NO) and scavenging free radicals, and completed the present invention accordingly.

### TECHNICAL MEANS FOR SOLVING THE PROBLEMS

To solve the problems that are described above, the present invention provides a cosmetic composition for skin whitening and wrinkle improvement comprising an adventitious root extract of *Centella asiatica* as an effective ingredient.

Furthermore, the present invention provides a pharmaceutical composition for external application on skin for skin whitening and wrinkle improvement comprising an adventitious root extract of *Centella asiatica* as an effective ingredient.

Furthermore, the present invention provides a pharmaceutical composition for prevention or treatment of melanin hyperpigmentation disorder comprising an adventitious root extract of *Centella asiatica* as an effective ingredient.

Still furthermore, the present invention provides a functional health food composition for prevention or amelioration of melanin hyperpigmentation comprising an adventitious root extract of *Centella asiatica* as an effective ingredient.

### ADVANTAGEOUS EFFECT OF THE INVENTION

The adventitious root extract of *Centella asiatica* has an excellent anti-inflammation and antioxidant activity without any cytotoxicity and also has an excellent effect of inhibiting the melanin pigmentation and enhancing the production of procollagen as a precursor of collagen, and thus it can be advantageously used as a cosmetic, a preparation for external application on skin, or the like for skin whitening and wrinkle improvement.

### BRIEF DESCRIPTION OF THE DRAWINGS

Fig.1 illustrates the result of determining the cytotoxicity of a water- or ethanol-extract of *Centella asiatica* or adventitious root of *Centella asiatica* for mouse macrophage. In the figure, nor represents normal cells without treatment; NMMA represents mono-methyl-L-arginine; and, indo represents indomethacin.
Fig. 2 illustrates the result of analyzing the production level of nitric oxide (NO) after treating LPS-treated mouse macrophage with a water- or ethanol-extract of *Centella asiatica* or adventitious root of *Centella asiatica.*
Fig. 3 illustrates the result of determining the cytotoxicity of a water- or ethanol-extract of *Centella asiatica* or adventitious root of *Centella asiatica* for mouse melanoma cells.
Fig. 4 illustrates the result of measuring the melanin level after treating mouse melanoma cells with a water- or ethanol-extract of *Centella asiatica* or adventitious root of *Centella asiatica.*
Fig. 5 illustrates the result of analyzing the change in production amount of MMP after treating UV-irradiated human fibroblast cells with a water- or ethanol-extract of *Centella asiatica* or adventitious root of *Centella asiatica.* In the figure, A shows the result from MMP-1 and B shows the result from MMP-3.
Fig. 6 illustrates the result of analyzing the production amount of procollagen after treating UV-irradiated human fibroblast cells with a water- or ethanol-extract of *Centella asiatica* or adventitious root of *Centella asiatica.*

### BEST MODE(S) FOR CARRYING OUT THE INVENTION

To achieve the object of the present invention, the present invention provides a cosmetic composition for skin whitening and wrinkle improvement comprising an adventitious root extract of *Centella asiatica* as an effective ingredient.

The adventitious root of *Centella asiatica* indicates adventitious root that is produced according to totipotency of plants when parts of wild *Centella asiatica* tissues are collected and introduced to a chamber in sterile state. If the adventitious root of *Centella asiatica* is subjected to seed culture for a certain period of time followed by main culture in a biological reactor, it can be produced in a large amount. In addition, as the adventitious root of *Centella asiatica* produced by the culture in chamber is basically free of residual pesticides (e.g., diazinon, DDT, and metalaxyl) or toxic components (e.g., lead and cadmium), it has advantages that it is safe from pest or virus infection which may occur during the growth process in nature.

With regard to the cosmetic composition for skin whitening and wrinkle improvement according to one embodiment of the present invention, the extract may be an extract of methanol, ethanol, propanol, butanol, or a mixture solvent thereof. It is preferably an ethanol extract and more preferably an extract obtained by room-temperature extraction for 22 to 26 hours by adding 60 to 80% (v/v) ethanol in an amount which is 8 to 12 times the weight of the dried adventitious root of *Centella asiatica,* but it is not limited thereto.

Furthermore, the adventitious root of *Centella asiatica* may comprise any one of the followings: extract obtained by an extraction treatment of the adventitious root of *Centella asiatica*; diluted solution or concentrate of the extract; dried product obtained by drying the extract; crude product; and purified product thereof.

As described herein, the expression "skin whitening" means suppression, inhibition, or amelioration of skin hyperpigmentation. Included in the skin hyperpigmentation disorder are freckles, dark spots, hyperpigmentation after exposure to UV ray, hyperpigmentation after inflammation, senile black spots, brown spots, and liver spots.

As described herein, the expression "wrinkle improvement" means suppression or inhibition of an occurrence of wrinkles on skin, or amelioration of wrinkles which have already occurred on skin. In the present invention, the occurrence of wrinkles is caused by reduced collagen, which may result from excessive oxidative stress or UV irradiation.

As the cosmetic composition of the present invention comprises, as an effective ingredient, an adventitious root extract of *Centella asiatica* which has a property of suppressing melanin production, a property of suppressing MMP production, and a property of promoting procollagen synthesis, it is a cosmetic composition having a skin whitening and wrinkle improvement function.

Furthermore, the adventitious root extract of *Centella asiatica* of the present invention is characterized by having more excellent property of inhibiting the nitric oxide (NO) production and scavenging free radicals than an extract of *Centella asiatica.*

With regard to the cosmetic composition according to one embodiment of the present invention, the cosmetic composition for skin whitening and wrinkle improvement can be a formulation that is selected from a group consisting of skin, skin softener, skin toner, astringent, lotion, milk lotion, moisture lotion, nutritional lotion, massage crème, nutritional crème, eye crème, moisture crème, hand crème, essence, nutritional essence, pack, cleansing foam, cleansing water, cleansing lotion, cleansing crème, body lotion, body cleanser, soap, and powder, but it is not limited thereto. The cosmetic composition in each formulation type above may comprise various bases and additives that are necessary and suitable for preparation of the formulation, and type and amount of those components can be easily selected by a person who is skilled in the art.

In a case in which the cosmetic composition of the present invention is a formulation like paste, crème, or gel, it is possible to use, as a carrier component, animal oil, plant oil, wax, paraffin, starch, tragacanth, cellulose derivatives, polyethylene glycol, silicone, bentonite, silica, talc, or zinc oxide.

In a case in which the cosmetic composition of the present invention is a formulation like solution or emulsion, a solvent, a dissolution agent, or an emulsifier is used as a carrier component, and examples thereof include water, ethanol, isopropanol, ethyl carbonate, ethyl acetate, benzyl alcohol, benzyl benzoate, propylene glycol, 1,3-butyl glycol oil, glycerol aliphatic ester, polyethylene glycol, and fatty acid ester of sorbitan.

In a case in which the cosmetic composition of the present invention is a formulation like suspension, it is possible to use, as a carrier component, a liquid phase diluent such as water, ethanol, or propylene glycol, a suspension agent such as ethoxylated isostearyl alcohol, polyoxyethlyene sorbitol ester, or polyoxyethylene sorbitan ester, microcrystalline cellulose, aluminum metahydroxide, bentonite, agar, or tragacanth.

In a case in which the cosmetic composition of the present invention is a formulation like a surfactant-containing cleanser, it is possible to use, as a carrier component, aliphatic alcohol sulfate, aliphatic alcohol ether sulfate, sulfosuccinic acid monoester, asethionate, imidazolinium derivatives, methyl taurate, sarcosinate, aliphatic amide ether sulfate, alkylamidobetaine, aliphatic alcohol, aliphatic glyceride, aliphatic diethanol amide, vegetable oil, lanolin derivatives, or ethoxylated glycerol aliphatic fatty acid ester.

In a case in which the cosmetic composition of the present invention is a formulation like powder or spray, it is possible to use, as a carrier component, lactose, talc, silica, aluminum hydroxide, calcium silicate, or polyamide powder, and, when it is spray, in particular, a propellant such as chlorofluoro hydrocarbon, propane-butane, or dimethyl ether may be additionally contained.

When the adventitious root extract of *Centella asiatica* of the present invention is prepared as a cosmetic product, it may be comprised at relatively high concentration in a wash-off type cosmetic like make-up remover and cleanser in which the effective ingredient stays on skin for a relatively short time. On the other hand, when the effective ingredient is supposed to stay on skin for a relatively long time, it is acceptable that the adventitious root extract of *Centella asiatica* is comprised at relatively low concentration compared to a wash-off type cosmetic.

The present invention also provides a pharmaceutical composition for external application on skin for skin whitening and wrinkle improvement comprising an adventitious root extract of *Centella asiatica* as an effective ingredient. In the pharmaceutical composition for external application on skin of the present invention, the adventitious root extract of *Centella asiatica* can be an ethanol extract of adventitious root of *Centella asiatica,* but it is not limited thereto.

The present invention also provides a pharmaceutical composition for prevention or treatment of melanin hyperpigmentation disorder comprising an adventitious root extract of *Centella asiatica* as an effective ingredient.

As described herein, the expression "melanin hyperpigmentation" means blackening or darkening of a certain area on skin, nail, or toe nail due to excessively increased melanin. Included in the melanin hyperpigmentation are freckles, senile spots, age spots, dark spots, brown or black spots, sunlight pigmentation, cyanic melasma, hyperpigmentation after use of pharmaceuticals, gravidic chloasma, and hyperpigmentation after inflammation that is caused by skin inflammation or wound resulting from friction burn or burn of skin, but it is not limited thereto.

The pharmaceutical composition of the present invention may comprise, other than the effective ingredient, a pharmaceutically acceptable carrier. Included in the carrier are lactose, dextrose, sucrose, sorbitol, mannitol, starch, acacia gum, calcium phosphate, alginate, gelatin, calcium silicate, microcrystalline cellulose, polyvinyl pyrrolidone, cellulose, water, syrup, methyl cellulose, methylhydroxy benzoate, propylhydroxy benzoate, talc, magnesium stearate, and mineral oil that are commonly used for having a preparation, but it is not limited thereto. The pharmaceutical composition of the present invention may additionally comprise, other than those described in the above, a lubricant, a wetting agent, a sweetener, a flavoring agent, an emulsifier, a suspending agent, and a preservative. When those components are added, the skin safety relating to use of a mixture, easiness for formulation, and safety of effective ingredients may be considered.

The pharmaceutical composition of the present invention may be administered either orally or parenterally, and, in case of parenteral administration, the administration can be made by topical application on skin, intravenous injection, subcutaneous injection, muscular injection, intraperitoneal injection, or transdermal administration. Considering that the pharmaceutical composition of the present invention is applied for treatment or prevention of a melanin hyperpigmentation disorder, it is preferably administered by topical application on skin.

The pharmaceutical composition of the present invention may be prepared in unit dose form by producing a preparation using a pharmaceutically acceptable carrier or vehicle according to a method that can be easily carried out a person who has common knowledge in the technical field to which the present invention pertains. Alternatively, it may be produced by introduction to a multi-dose container. In that case, the formulation can be prepared in any formulation selected from injection solution, cream, patch, spray, soft ointment, plaster, lotion, liniment, pasta preparation, and cataplasma preparation, and it may additionally comprise a dispersant or a stabilizer.

By comprising the adventitious root extract of *Centella asiatica* in an effective amount, the pharmaceutical composition of the present invention can provide a desired effect of skin whitening and wrinkle improvement. As described herein, the expression "effective amount" means an amount of the extract which can exhibit the effect of suppressing pigmentation or improving skin wrinkles. The effective amount of an adventitious root extract of *Centella asiatica* to be contained in the composition of the present invention may vary depending on a product type of the composition, a method for applying the adventitious root extract of *Centella asiatica* to skin, retention time of the adventitious root extract of *Centella asiatica* on skin, or the like. For example, when the adventitious root extract of *Centella asiatica* is produced as a pharmaceutical product, the adventitious root extract of *Centella asiatica* may be comprised at higher concentration compared to a case in which it is prepared as a cosmetic product to be applied regularly on skin. As such, the daily dose is, based on the amount of adventitious root extract of *Centella asiatica,* 0.1 to 100 mg/kg, preferably 30 to 80 mg/kg, and more preferably 50 to 60 mg/kg, and it can be administered 1 to 6 times per day. Preferred dosage of the pharmaceutical composition for external application on skin for skin whitening and wrinkle improvement comprising an adventitious root extract of *Centella asiatica* as an effective ingredient may vary depending on conditions and bodyweight of a patient, severeness of a disease, dosage form, administration route, and administration period, but it can be suitably selected by a person who is skilled in the art.

The present invention also provides a functional health food composition for prevention or amelioration of melanin hyperpigmentation comprising an adventitious root extract of *Centella asiatica* as an effective ingredient.

When the functional health food composition of the present invention is used as a food additive, the functional health food composition may be directly added or used in combination with other food product or food component, and it can be suitably used according to a common method. The mixing amount of the effective ingredient can be suitably set depending on the purpose of use (e.g., prevention or amelioration). In general, for producing a food product or a beverage, the functional health food composition of the present invention is added in an amount of 15 parts by weight or less, and preferably in an amount of 10 parts by weight or less relative to raw materials. However, when it is consumed for a long period of time for health enhancement, the amount can be less than the aforementioned amount. By having no problem in terms of safety, the effective ingredient may be also used in an amount that is higher than the aforementioned amount.

Type of the functional health food composition is not particularly limited. Examples of a food product to which the functional health food composition may be added include meat products, sausages bread, chocolate, candies, snacks, cookies, pizza, ramen, other noodles, gums, dairy products including ice cream, various soups, beverages, tea drinks, alcohol beverages and vitamin complexes, and all food products in general sense are included therein.

Furthermore, the functional health food composition of the present invention can be produced as a food product, in particular, a functional food product. The functional food product of the present invention comprises a component that is generally added for producing a food product, and it includes proteins, carbohydrates, lipids, nutrients, and flavoring agents, for example. When it is prepared as a drink preparation, for example, natural carbohydrates or a flavoring agent may be added as an additional ingredient other than the effective ingredient. The natural carbohydrates are preferably monosaccharides (for example, glucose and fructose), disaccharides (for example, maltose and sucrose), oligosaccharides, polysaccharides (for example, dextrin and cyclodextrin), or sugar alcohols (for example, xylitol, sorbitol, and erythritol). As for the flavoring agent, a natural flavoring agent (for example, taumatin and stevia extract) and a synthetic flavoring agent (for example, saccharine and aspartame) can be used.

The functional health food composition may comprise, other than those described above, various nutritional agents, vitamins, electrolytes, flavors, coloring agent, pectinic acid and salt thereof, alginic acid and salt thereof, organic acids, protective colloidal thickening agent, pH regulating agent, stabilizing agent, preservative, glycerin, alcohol, and carbonating agent used for carbonated drink. Ratio of those components for addition is, although not particularly critical, generally selected from a range of from 0.01 to 0.1 part by weight relative to 100 parts by weight of the functional health food composition of the present invention.

Hereinbelow, the present invention is explained in greater detail in view of the Examples. However, it is evident that the following Examples are given only for exemplification of the present invention and by no means the present invention is limited to the following Examples.

### EXAMPLES

### Preparation example 1. Preparation of adventitious root extract of Centella asiatica

### (1) Preparation of 70% ethanol extract of adventitious root of Centella asiatica

To 10 g of dried adventitious root of *Centella asiatica* (prepared by Center for Development of Advanced Horticultural Technology, Chungbuk National University), 100 ml of 70% (v/v) aqueous ethanol solution were added. After that, room-temperature extraction was carried out for 24 hours followed by filtration through 0.45 µm filter, and then concentration under reduced pressure and drying under hot air were carried out.

### (2) Preparation of water extract of adventitious root of Centella asiatica

To 10 g of dried adventitious root of *Centella asiatica,* 100 ml of purified water were added. After that, room-temperature extraction was carried out for 24 hours followed by filtration through 0.45 µm filter, and then concentration under reduced pressure and drying under hot air were carried out.

### (3) Preparation of 70% ethanol extract of Centella asiatica

To 10 g of dried leaves of *Centella asiatica* (purchased from Pierre Favre (France)), 100 ml of 70% (v/v) ethanol solution were added. After that, room-temperature extraction was carried out for 24 hours followed by filtration through 0.45 µm filter, and then concentration under reduced pressure and drying under hot air were carried out.

### (4) Preparation of water extract of Centella asiatica

To 10 g of dried leaves of *Centella asiatica,* 100 ml of purified water were added. After that, room-temperature extraction was carried out for 24 hours followed by filtration through 0.45 µm filter, and then concentration under reduced pressure and drying under hot air were carried out.

### Example 1. Measurement of cytotoxicity

Cytotoxicity was measured for the water or ethanol extract of adventitious root of *Centella asiatica* or *Centella asiatica,* which has been prepared in the above Preparation example 1. Mouse macrophage (RAW264.7) induced to have an inflammation response by 0.5 µg/ml LPS (lipopolysaccharide) was added to a 96-well plate at 2 × 10⁵ cells/well. After culture for 24 hours, the cells were treated with NMMA (50 µg/ml), indomethacin (2 ng/ml), or each extract sample (100 or 200 µg/ml) and cultured for 48 hours. Then, an MTT assay was carried out to measure the cell viability.

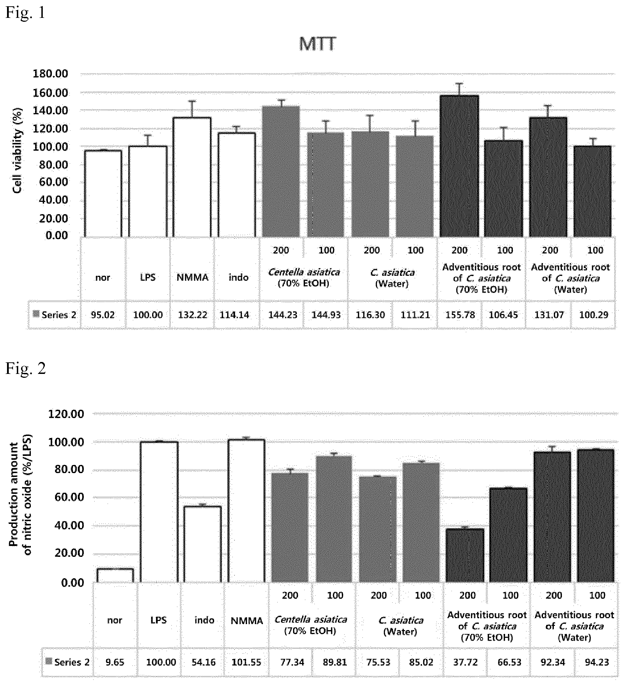

As a result, as it is shown in Fig. 1, it is found that cytotoxicity is not exhibited by an extract of *Centella asiatica* or an adventitious root extract of *Centella asiatica.*

### Example 2. Analysis of anti-inflammation activity

RAW264.7 cells which have been induced to have an inflammation response by LPS in the same manner as above were added to a 96-well plate. After culture for 24 hours, the cells were treated with an extract sample of *Centella asiatica* or adventitious root of *Centella asiatica* and cultured for 48 hours. Then, by using the Griess reagent, the production amount of NO was measured.

As a result, as it is shown in Fig. 2, it was found that the NO production is not inhibited in a group treated with NMMA (Mono-methyl-L-arginine) but the enhanced NO production, which is caused by the LPS treatment, has decreased in an indomethacin treatment group (indo). In addition, it was also found that the NO production is suppressed in a group treated with 70% ethanol extract of adventitious root of *Centella asiatica.* It was found that the water extract of adventitious root of *Centella asiatica* and water extract and ethanol extract of *Centella asiatica,* in which the same sample is used, all exhibit no activity of suppressing the NO production.

### Example 3. Analysis of antioxidant activity

The antioxidant activity of 70% ethanol extract of *Centella asiatica* or adventitious root of *Centella asiatica,* which has been prepared in Preparation example 1, was analyzed. In brief, after mixing 190 µl of 0.1 mM DPPH (2,2-diphenyl-1-picrylhydrazyl) with 10 µl of the extract sample, they were allowed to react for 30 minutes in a dark room. Then, by measuring the absorbance at 517 nm, the radical scavenging activity was evaluated.

As a result, it was found that the 70% ethanol extract of adventitious root of *Centella asiatica* exhibits a stronger DPPH radical scavenging activity than the 70% ethanol extract of *Centella asiatica,* as it is shown in the following Table 1.

**[Table 1]**

| DPPH Radical Scavenging Activity | | |
|---|---|---|
| | Ethanol extract of adventitious root of *Centella asiatica* | Ethanol extract of *Centella asiatica* |
| IC₅₀ (µg/ml) | 18.5 ± 4.6 | 184.0 ± 9.8 |

### Example 4. Evaluation of skin whitening effect

In order to evaluate the effect of suppressing melanin synthesis by the extract prepared in Preparation example 1, mouse melanoma cells (B-16 melanoma cell) were cultured using a DMEM medium containing 10% FBS, added to a 6-well plate at 3 × 10⁵ cells/well followed by culture for 24 hours. Then, the cells were treated with kojic acid at a concentration of 50 µg/ml or with each sample at a concentration of 50, 100, or 200 µg/ml followed by culture for 3 days. After the culture, the cells were washed 2 times with PBS and treated with trypsin to release the cells from the culture container. The resultant was centrifuged to obtain cell precipitates. To the extracted melanin, 1 ml of 1 N sodium hydroxide solution was added and boiled for about 10 minute to dissolve the melanin. Then, the absorbance at 400 nm was measured. The melanin amount was obtained from a standard curve which has been established by using synthetic melanin, and the melanin amount of test group was calculated in terms of the percentage relative to the melanin amount of control group. Furthermore, toxicity of the extract for B-16 cells was analyzed by an MTT assay.

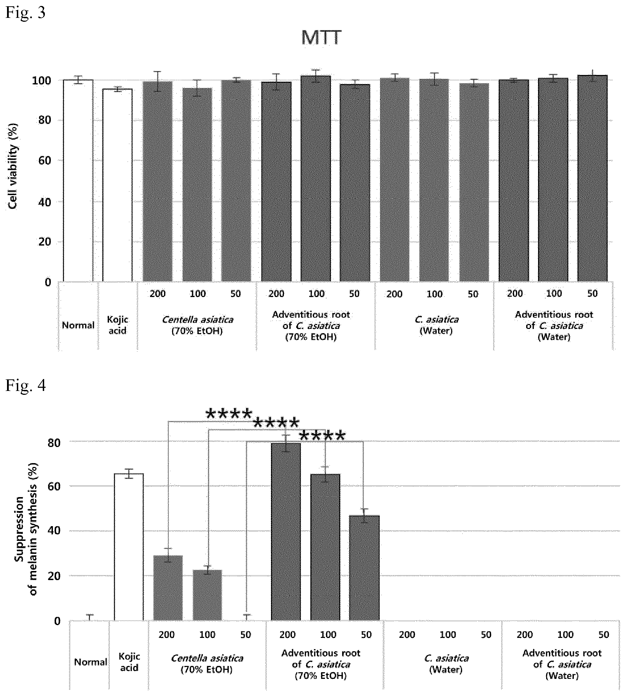

As a result, both the water extract and 70% ethanol extract of *Centella asiatica* or adventitious root of *Centella asiatica* did not exhibit any cytotoxicity (Fig. 3), and the water extract of *Centella asiatica* or adventitious root of *Centella asiatica* has no activity of inhibiting melanin synthesis. However, the 70% ethanol extract of adventitious root of *Centella asiatica* showed the effect of suppressing melanin synthesis, i.e., 78.8%, 65.1%, or 46.8% suppression by the treatment at concentration condition of 200, 100, or 50 µg/ml, while the 70% ethanol extract of *Centella asiatica* showed the effect of suppressing melanin synthesis, i.e., 29.1%, 22.5%, or 0% suppression by the treatment at concentration condition of 200, 100, or 50 µg/ml, and thus it was found that the ethanol extract of adventitious root of *Centella asiatica* has a significantly higher effect of suppressing melanin synthesis compared to the ethanol extract of *Centella asiatica* (Fig. 4).

Furthermore, the tyrosinase inhibitory activity of ethanol extract of adventitious root of *Centella asiatica* and ethanol extract of *Centella asiatica* was analyzed. In brief, 220 µl of 0.1 M sodium phosphate buffer and 20 µl of 200 µl/ml extract sample were admixed with each other, and added with 40 µl of 1.5 mM L-tyrosine and 20 µl of mushroom tyrosinase which has been prepared at 1,500 units/ml. After the reaction for 15 minutes at 37 °C, the absorbance at 490 nm was measured to evaluate the amount of dopachrome that is produced. As a result, as it is shown in the following Table 2, it was found that the tyrosinase inhibitory activity of the 70% ethanol extract of adventitious root of *Centella asiatica* shows no significant difference compared to the 70% ethanol extract of *Centella asiatica.*

Based on the results that are described above, it is recognized that the 70% ethanol extract of adventitious root of *Centella asiatica* has an excellent effect of inhibiting the synthesis of melanin pigment, but it is based on a mechanism other than the inhibitory effect on tyrosinase enzyme activity.

**[Table 2]**

| Tyrosinase inhibitory activity | | |
|---|---|---|
| | Ethanol extract of adventitious root of *Centella asiatica* | Ethanol extract of *Centella asiatica* |
| Tyrosinase inhibitory activity (%) | 26.6 ± 1.7 | 30.6 ± 11.5 |

### Example 5. Evaluation of wrinkle improvement

In order to determine the effect of wrinkle improvement by the extract prepared in Preparation example 1, the effect of inhibiting the synthesis of enzyme-1 (matrix metalloprotease-1, MMP-1) and MMP-3 proteins and the effect of promoting the synthesis of procollagen protein were analyzed. In brief, to a 40 mm cell culture dish, DMEM culture medium (2 ml) was added, inoculated with human fibroblast cells at a concentration of 1.2 × 10⁵ cells/dish, and cultured for 24 hours in 5% carbon dioxide environment at 37 °C. After that, the cells were irradiated with UVB at conditions of 144 mJ/cm². Thereafter, the medium was replaced with a fresh medium containing each extract sample followed by culture for 3 days. The cell culture solution was then collected, centrifuged for 5 minutes at 7500 rpm, 4°C to collect the supernatant. Then, a change in the expression amount of MMP-1 and MMP-3 proteins was determined by using Human total MMP-1/3 kit (R&D Systems, Inc., Minneapolis, MN, USA).

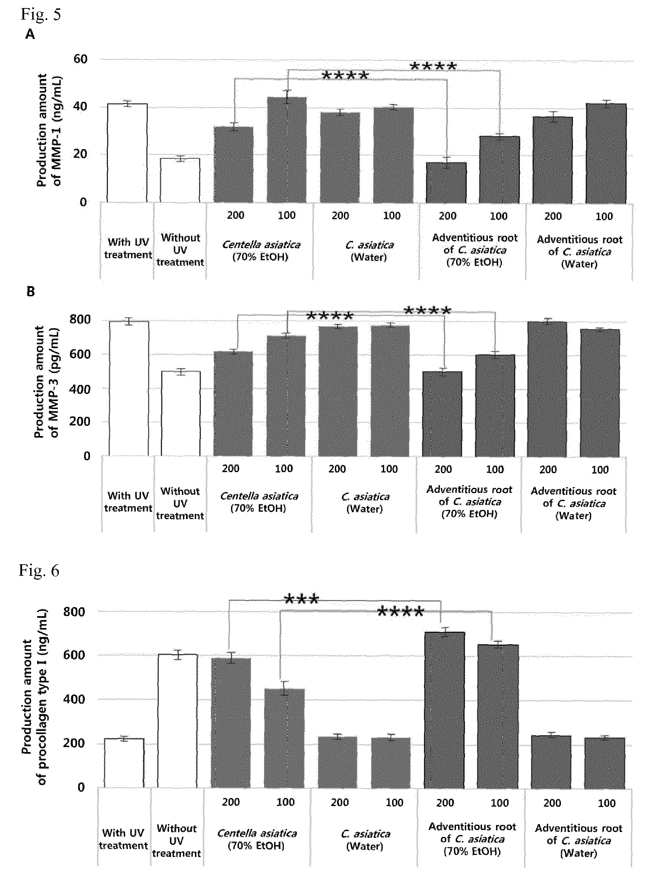

As a result, the human fibroblast cells obtained after UV irradiation showed the MMP-1 protein production amount which has increased by 2 times or so compared to the group without having any UV treatment, and the increased production amount of MMP-1, which is caused by UV irradiation, has decreased by 58.6% and 23.1%, respectively, according to a treatment with the ethanol extract of adventitious root of *Centella asiatica* or the ethanol extract of *Centella asiatica* at a concentration of 200 µg/ml. In particular, it was found that the ethanol extract of adventitious root of *Centella asiatica* showed the same level as the group without any UV treatment, and, based on those results, it was found that the ethanol extract of adventitious root of *Centella asiatica* inhibits highly effectively the production of MMP-1 (Fig. 5A). With regard to the production amount of MMP-3 protein, it was also observed that the ethanol extract of adventitious root of *Centella asiatica* or the ethanol extract of *Centella asiatica* has the effect of inhibiting the MMP-3 protein production, 36.9% and 22.3%, respectively, at a concentration of 200 µg/ml (Fig. 5B). In particular, in a case in which the extraction is made with water even with use of the same adventitious root of *Centella asiatica,* the aforementioned effect of inhibiting the MMP protein production is not observed. Based on this result, it was recognized that, by effectively inhibiting the production of MMP-1 and MMP-3 proteins as a cause of wrinkle formation, the ethanol extract of adventitious root of *Centella asiatica* of the present invention can be effectively used for prevention and improvement of skin wrinkles.

Furthermore, to examine the influence of each extract of the present invention on collagen production, cells were prepared at the same conditions as the above test conditions for determining the inhibitory effect on MMP protein production, and a change in the type I procollagen protein expression amount in cell culture solution irradiated with UVB was examined by using Procollagen Type I C Peptide EIA kit (Takara, Shiga, Japan).

As a result, it was found that, compared to the group not treated with UV ray, the human fibroblast cells obtained after UV irradiation show a significantly reduced production amount of type 1 procollagen protein, but the reduced production amount of type 1 procollagen protein has increased by 319.4% and 266.2%, respectively, under conditions at which the cells are treated with an ethanol extract of adventitious root of *Centella asiatica* or an ethanol extract of *Centella asiatica* at 200 µg/ml (Fig. 6). Like the test for determining the property of inhibiting MMP protein production, the water extract of adventitious root of *Centella asiatica* showed no effect of enhancing the procollagen production. Based on the above results, it was recognized that the 70% ethanol extract of adventitious root of *Centella asiatica* of the present invention has an effect of preventing effectively the wrinkle formation and an effect of improving formed wrinkles based on the activity of promoting the production of type 1 procollagen and inhibiting the expression of MMP-1 and MMP-3 as an enzyme for degrading collagen.

### <Formulation example 1> Preparation of skin softener (skin)

As described in the followings, a skin softener (skin) comprising the ethanol extract of adventitious root of *Centella asiatica,* which has been obtained from Preparation example 1-(1), was prepared according to the composition of the following table by following a common method.

**[Table 3]**

| Composition of skin softener blend | |
|---|---|
| Ingredient | Content (unit: % by weight) |
| Ethanol extract of adventitious root of *Centella asiatica* | 2.0 |
| Glycerin | 5.0 |
| 1.3-Butylene glycol | 3.0 |
| PEG 1500 | 1.0 |
| Allantoin | 0.1 |
| DL-Panthenol | 0.3 |
| EDTA-2Na | 0.02 |
| Benzophenone-9 | 0.04 |
| Sodium hyaluronate | 5.0 |
| Ethanol | 10.0 |
| Octyldocedeth-16 | 0.2 |
| Polysorbate 20 | 0.2 |
| Preservative, fragrance, and pigment | trace amount |
| Distilled water | residual amount |
| Total | 100 |

### <Formulation example 2> Preparation of nutritional cosmetic toner (lotion)

As described in the followings, a nutritional cosmetic toner comprising the ethanol extract of adventitious root of *Centella asiatica,* which has been obtained from Preparation example 1-(1), was prepared according to the composition of the following table by following a common method.

**[Table 4]**

| Composition of nutritional cosmetic toner (lotion) | |
|---|---|
| Ingredient | Content (unit: % by weight) |
| Ethanol extract of adventitious root of *Centella asiatica* | 2.0 |
| Glyceryl stearate SE | 1.5 |
| Stearyl alcohol | 1.5 |
| Lanolin | 1.5 |
| Polysorbate 60 | 1.3 |
| Sorbitan stearate | 0.5 |
| Hydrogenated vegetable oil | 1.0 |
| Mineral oil | 5.0 |
| Squalane | 3.0 |
| Trioctanoin | 2.0 |
| Dimethicone | 0.8 |
| Tocopherol acetic acid | 0.5 |
| Carboxyvinyl polymer | 0.12 |
| Glycerin | 5.0 |
| 1,3-Butylene glycol | 3.0 |
| Sodium hyaluronate | 5.0 |
| Triethanolamine | 0.12 |
| Preservative, fragrance, and pigment | trace amount |
| Distilled water | residual amount |
| Total | 100 |

### <Formulation example 3> Preparation of nutritional crème

As described in the followings, a nutritional crème comprising the ethanol extract of adventitious root of *Centella asiatica,* which has been obtained from Preparation example 1-(1), was prepared according to the composition of the following table by following a common method.

**[Table 5]**

| Composition of nutritional crème | |
|---|---|
| Ingredient | Content (unit: % by weight) |
| Ethanol extract of adventitious root of *Centella asiatica* | 2.0 |
| Lipophilic monostearic acid glycerin | 2.0 |
| Cetearyl alcohol | 2.2 |
| Stearic acid | 1.5 |
| Bee wax | 1.0 |
| Polysorbate 60 | 1.5 |
| Sorbitan stearate | 0.6 |
| Hydrogenated vegetable oil | 1.0 |
| Squalane | 3.0 |
| Mineral oil | 5.0 |
| Trioctanoin | 5.0 |
| Dimethicone | 1.0 |
| Sodium magnesium silicate | 0.1 |
| Glycerin | 5.0 |
| Betaine | 3.0 |
| Triethanol | 1.0 |
| Sodium hyaluronate | 4.0 |
| Preservative, fragrance, and pigment | trace amount |
| Distilled water | residual amount |
| Total | 100 |

### <Formulation example 4> Preparation of essence

As described in the followings, an essence comprising the ethanol extract of adventitious root of *Centella asiatica,* which has been obtained from Preparation example 1-(1), was prepared according to the composition of the following table by following a common method.

**[Table 6]**

| Composition of essence | |
|---|---|
| Ingredient | Content (unit: % by weight) |
| Ethanol extract of adventitious root of *Centella asiatica* | 2.0 |
| Glycerin | 10.0 |
| Betaine | 5.0 |
| PEG 1500 | 2.0 |
| Allantoin | 0.1 |
| DL-Panthenol | 0.3 |
| EDTA-2Na | 0.02 |
| Benzophenone-9 | 0.04 |
| Hydroxyethyl cellulose | 0.1 |
| Sodium hyaluronate | 8.0 |
| Carboxyvinyl polymer | 0.2 |
| Triethanol | 0.18 |
| Octyl dodecanol | 0.3 |
| Octyldocedeth-16 | 0.4 |
| Ethanol | 6.0 |
| Preservative, fragrance, and pigment | trace amount |
| Distilled water | residual amount |
| Total | 100 |

## Claims

1. A cosmetic composition for skin whitening and wrinkle improvement comprising an adventitious root extract of *Centella asiatica* as an effective ingredient.

2. The cosmetic composition according to Claim 1, wherein the extract is extracted by using lower alcohol with carbon atom number of 1 to 4 or a mixed solvent thereof.

3. The cosmetic composition according to Claim 1, wherein the composition inhibits production of melanin and matrix metalloprotease but promotes production of procollagen.

4. The cosmetic composition according to Claim 1, wherein the composition has an anti-inflammation and antioxidant activity.

5. The cosmetic composition according to Claim 1, wherein the composition is any one formulation selected from skin, skin softener, skin toner, astringent, lotion, milk lotion, moisture lotion, nutritional lotion, massage crème, nutritional crème, eye crème, moisture crème, hand crème, essence, nutritional essence, pack, cleansing foam, cleansing water, cleansing lotion, cleansing crème, body lotion, body cleanser, soap, and powder.

6. A pharmaceutical composition for external application on skin for skin whitening and wrinkle improvement comprising an adventitious root extract of *Centella asiatica* as an effective ingredient.

7. A pharmaceutical composition for prevention or treatment of melanin hyperpigmentation disorder comprising an adventitious root extract of *Centella asiatica* as an effective ingredient.

8. The pharmaceutical composition for prevention or treatment of melanin hyperpigmentation disorder according to Claim 7, wherein the melanin hyperpigmentation disorder is freckles, senile spots, age spots, dark spots, brown or black spots, sunlight pigmentation, cyanic melasma, hyperpigmentation after use of pharmaceuticals, gravidic chloasma, or hyperpigmentation after inflammation that is caused by skin inflammation or wound resulting from friction burn or burn of skin.

9. The pharmaceutical composition for prevention or treatment of melanin hyperpigmentation disorder according to Claim 7, wherein the composition is prepared in any formulation selected from injection solution, cream, patch, spray, soft ointment, plaster, lotion, liniment, pasta preparation, and cataplasma preparation.

10. A functional health food composition for prevention or amelioration of melanin hyperpigmentation comprising an adventitious root extract of *Centella asiatica* as an effective ingredient.
